Europäisches Patentamt

European Patent Office

Office européen des brevets

(11) Publication number: **0 303 461 B1**

(12) **EUROPEAN PATENT SPECIFICATION**

(45) Date of publication of patent specification: **30.12.92**  (51) Int. Cl.⁵: **A61K  7/32, A61K 7/00**

(21) Application number: **88307394.2**

(22) Date of filing: **10.08.88**

(54) **Skin treatment composition.**

| | |
|---|---|
| (30) Priority: **12.08.87 GB 8719091** | (73) Proprietor: **UNILEVER PLC**<br>**Unilever House Blackfriars P.O. Box 68**<br>**London EC4P 4BO(GB)** |
| (43) Date of publication of application:<br>**15.02.89 Bulletin  89/07** | (84) Designated Contracting States:<br>**GB** |
| (45) Publication of the grant of the patent:<br>**30.12.92 Bulletin  92/53** | (73) Proprietor: **UNILEVER N.V.**<br>**Weena 455**<br>**NL-3013 AL Rotterdam(NL)** |
| (84) Designated Contracting States:<br>**AT BE CH DE ES FR GB GR IT LI NL SE** | (84) Designated Contracting States:<br>**BE CH DE ES FR GR IT LI NL SE AT** |
| (56) References cited:<br>**AU-A- 437 703**<br>**FR-A- 2 033 294** | (72) Inventor: **Wight, Gordon Robert**<br>**4 Vanderbyl Avenue**<br>**Spital Wirral Merseyside L62 2AP(GB)** |
| | (74) Representative: **Elliott, Peter William et al**<br>**Unilever plc, Patent Division Colworth House**<br>**Sharnbrook**<br>**Bedford MK44 1LO(GB)** |

Rank Xerox (UK) Business Services

## Description

### FIELD OF INVENTION

The invention relates to deodorant and antiperspirant compositions suitable for topical application to human skin, which are adapted to generate a sensory signal, and/or to deliver another benefit agent, such as a deodorant, antiperspirant or anticholinergic active substance, on contact with skin moisture. Those compositions that generate a sensory signal are intended to indicate to the user that they are responding to moisture on the skin, and therefore increase personal confidence in their intended function.

### BACKGROUND AND PRIOR ART

It is well known that body malodour problems are at least partly associated with perspiration, and to combat this problem, aerosol, roll-on and stick products having deodorant properties have been developed for topical application to human skin, particularly to the underarm.

Antiperspirant products generally contain an astringent metallic aluminium salt, or the like, which is intended to reduce the amount of perspiration accumulating at the skin surface, and deodorant products generally contain an antibacterial substance for reducing or eliminating body malodour which develops in the presence of skin moisture. Both types of product can contain ethanol which not only functions as a vehicle for antiperspirant and deodorant active substances, but can act as a deodorant in its own right.

It is important that the user of deodorant and antiperspirant products is confident that body malodour and perspiration will be significantly reduced or eliminated following their use, and to this end, it would be advantageous to provide a sensory response to signal that such products really are working.

With this in mind, The Mennen Company in GB 1 275 969 proposed the use of spray-dried deodorant capsules of dextrin, gum arabic or film-forming polypeptides containing perfume for use in aerosol deodorants, the capsules being soluble in water but insoluble in and impermeable to aerosol propellants. When preparing an aerosol deodorant, the deodorant capsules, for example of gum arabic, and containing perfume and hexachlorophene, are mixed with the remaining components including aerosol propellant and a suspending agent, to provide perspiration-responsive aerosol deodorant. There are, however, clear instructions in the Mennen reference that the "final charge should not contain a substantial amount of alcohol - present in virtually all other aerosol deodorants - since alcohol (ie., ethanol or the like) ordinarily would leach the encapsulate from the capsules and thereby frustrate their main purpose."

International Flavors & Fragrances in US 4 428 869 are concerned with encapsulating fragrance oils in, for example, gum acacia for use in hydroalcoholic systems, in which the water-alcohol mixtures can contain from 80 to 95% alcohol.

FrA 2033291 (L'Oreal) discloses deodorant compositions containing 0.5% of moisture sensitive microcapsules having walls of ethyl cellulose, and having an average particle size of $30\mu$m, the formulation containing 94% alcohol. The microcapsules can contain hexachlorophene (see example 3).

In our endeavours to develop alcohol-containing antiperspirant and deodorant products for topical application to skin, which are capable of generating a sensory response to signal the advent of moisture at the skin surface, and/or delivering another benefit agent triggered by the same mechanism, we have discovered contrary to expectation, particularly in the light of the teaching of the Mennen reference, that perfume encapsulated by spray drying with gum acacia (also known as gum arabic or gum senegal) is remarkably resistant to leaching with ethanol when tested using continous extraction with anhydrous ethanol at room temperature over a long period of time. It is believed that this extraordinary stability of the capsule in ethanol is due to the use of ethanol which is virtually anhydrous, whereas it is likely that the instability reported in the Mennen reference was due to the small but significant amount of water which is commonly present in alcohol as used in the deodorant industry, unless stringent precautions are taken to maintain the ethanol in an anhydrous state, or nearly so. It is noted that although the Mennen reference is silent on this point, the presence of a small amount, for example up to about 5% by weight, of water in the ethanol employed by them, would be sufficient to disrupt their capsules such that the perfume and bactericide is easily leached by ethanol.

A similar effect would result when using the hydroalcoholic systems taught by International Flavours & Fragrancies, referred to earlier, which also contain at least 5% water.

This is the only logical explanation of Mennen's cautionary instructions that the "final charge should not contain a substantial amount of alcohol."

### DEFINITION OF THE INVENTION

Accordingly, the invention provides an alcoholic composition suitable for topical application to human skin which comprises:

i) from 0.1 to 20% by weight of moisture-sensitive capsules; and

ii) from 10 to 99.9% by weight of an alcohol chosen from $C_{2-6}$ alkanols and mixtures thereof;

the composition comprising no more than 3% by weight of water;

the moisture sensitive capsules having an average particle size of not greater than $100\mu$m, and comprising a non-aqueous substance encapsulated by a polymer, the capsules being:

(a) resistant to extraction with alcohol such that not more that 15% by weight of the non-aqueous substance originally present in the capsules is leached from the capsules during continuous extraction of the intact capsules for a period of 30 days at 20°C with ethanol containing not more than 1% by weight of water; and

(b) sensitive to moisture such that on contact with water at the skin surface, the capsules release the encapsulated non-aqueous substance.

DISCLOSURE OF THE INVENTION

The Moisture Sensitive Capsules

The alcoholic composition according to the invention comprises moisture-sensitive capsules, in which a non-aqueous substance is encapsulated by a special polymer that forms a coating resistant to disruption by alcohol, provided the composition comprises no more than 3% by weight, yet sensitive to moisture as herein defined.

The Special Polymer

The special polymer from which the capsules are formed is preferably a polysaccharide. Examples of suitable polysaccharides are dextrins, especially low-viscosity dextrins, including maltodextrins.

A particularly preferred example of a low-viscosity dextrin is one which, as a 50% dispersion in water has a viscosity at 25°C, using a Brookfield Viscometer fitted with an "A" type T-Bar rotating at 20 rpm in helical mode, of 330 ± 20 mPas. This dextrin is known as Encapsul® 855, (also described as Encapsol 855) and is available from National Starch and Chemicals Ltd.

A further example of a polysaccharide that can be used to form the moisture-sensitive capsules is gum acacia.

The Non-Aqueous Substance

The moisture-sensitive capsules contain a non-aqueous substance, preferably one which is capable of providing a sensory response following disruption of the capsules in the presence of moisture, particularly with perspiration or other skin moisture.

Examples of non-aqueous substances which are capable of providing a sensory response on being released from the capsules when contacted with moisture include the following:

(i) Perfumes, which give rise to an olfactory response in the form of a fragrance, and deodorant perfumes, such those described in US patent 4 278 658 (Lever Brothers & Co) which in addition to providing a fragrance response, can also reduce body malodour;

(ii) Skin coolants, such as menthol, menthyl lactate, menthyl pyrrolidone carboxylate N-ethyl-p-menthane-3-carboxamide and other derivatives of menthol, which give rise to a tactile response in the form of a cooling sensation on the skin; and

(iii) Emollients such as isopropylmyristate, silicone oils, mineral oils and vegetable oils which give rise to a tactile response in the form of an increase in skin lubricity.

Examples of non-aqueous substances that can provide another skin benefit are the following:

(iv) Deodorants other than perfumes, whose function is to reduce the level of or eliminate microflora at the skin surface, especially those responsible for the development of body malodour, examples of which are:

2,4,4'-trichloro-2'-hydroxydiphenyl ether (also known as Irgasan DP300 or Triclosan), cetyltrimethylammonium bromide, cetylpyridinium chloride, benzethonium chloride, di-isobutyl phenoxethoxyethyl dimethylbenzylammonium chloride, N-alkylpyridinium chloride, N-cetylpyridinium bromide, sodium-N-lauroyl sarcosine, zinc phenyl sulphonate, farnesol and ethyl lactate. Precursors of deodorants other than perfume can also be employed.

3

(v) Antiperspirant actives, whose function is to reduce or eliminate the appearace of perspiration at the skin surface, examples of which are:

aluminium chloride; aluminium sulphate; aluminium chlorohydrate; basic aluminium bromide, zirconyl chloride; zirconyl hydroxide; zirconyl chorohydrate; complexes of aluminium hydroxide, zirconyl chloride and aluminium chlorohydrate; complexes of aluminium hydroxide, zirconyl chlorohydrate, and aluminium chlorohydrate; complexes of dihydroxyaluminium glycinate, zirconyl chloride and/or zirconyl chlorohydrate and aluminium chlorohydrate; complexes of zirconyl chloride and/or zirconyl chlorohydrate and aluminium chlorohydrate; complexes of zirconyl chloride and/or zirconyl chlorohydrate with aluminium chlorohydrate and an amino acid, such as glycine; and mixtures of two or more of the above. Most preferred materials are those which have a high antiperspirant efficacy. One class of such materials are the aluminium zirconium chlorohydrate complexes. examples of which are aluminium zirconium trichlorohydrate; aluminium zirconium tetrachlorohydrate and alumium zirconium pentachlorohydrate (these are CTFA generic names). These compounds may be combined with glycine to give for example the compounds known under the CTFA generic names aluminium zirconium trichlorohydrex-GLY and aluminium zirconium tetrachlorohydrex-GLY. Methods for preparing aluminium zirconium chlorohydrates are described in US Patents Nos. 4 028 390 (Armour) and 3 792 068 (Procter & Gamble). Suitable aluminium zirconium chlorohydrate powders for use in the antiperspirant compositions of this invention are available from the Reheis Chemical Company under the trade names REZAL® 36 GP and REZAL® 67P (REZAL is trade mark) and from Dow Corning under the trade names Dow Corning AZG-368, AZG-369 & AZG-370. Other preferred antiperspirant active materials of high efficacy are the special active forms of basic aluminium chloride which have a particular distribution of polymeric species in aqueous solution and obtainable by procedures described in US Patent No. 4 359 456 (Gosling et al). Similar processes for making highly active forms of aluminium chlorohydrate involving the ageing of aluminium chlorohydrate in an aqueous medium are described in British Patent Specification No. 2 144 992 (Gillette). The antiperspirant agent may also be a urea or glycine complex of aluminium chlorohydrate prepared as described in European Patent No. 6738 (Unilever) and British Patent No. 1 597 497 (Unilever).

(vi) Anticholinergic actives, whose function is to reduce or eliminate the generation of perspiration before it reaches the skin surface, examples of which are scopolamine derivatives, such as scopolamine hydrobromide and esters thereof, such as benzoyl scopolamine hydrobromide.

Mixtures of non-aqueous substances, such as those referred to herein, can also be employed. Examples of such mixtures include:

perfume + non-perfumery deodorant

perfume + anticholinergic active

perfume + anticholinergic + antiperspirant actives

perfume + coolant

The weight ratio of special polymer forming the capsular material of the capsules to the non-aqueous substance is preferably from 1:10 to 100:1, most preferably from 1:4 to 9:1.

Formation of the moisture sensitive capsules

The moisture-sensitive capsules can be formed, for example, by preparing an emulsion of water, the special polymer and the non-aqueous substance, together with any other materials dissolved or dispersed therein, which are required to be included in the capsules. The emulsion is then spray-dried according to conventional technology to form capsules of the polymer containing the encapsulate.

By way of example, the moisture sensitive capsules can be prepared by forming an emulsion comprising from 20 to 40%, preferably from 23 to 36% by weight of the special polymer and from 5 to 30%, preferably from 7 to 29% by weight of the non-aqueous substance, together with from 40 to 70%, preferably from 43 to 65% by weight water, and then spray-drying this emulsion.

Moisture-sensitive capsules so prepared will accordingly comprise approximately from 10 to 60% by weight of non-aqueous substance encapsulated by from 40 to 90% by weight of special polymer.

Properties of the moisture-sensitive capsules

The capsules so formed are capable of dissolving in or becoming permeable on contact with moisture, particularly that at the skin surface, to release the encapsulated non-aqueous substance, but are surprisingly resistant to contact with an ethanolic composition containing no more than 3% by weight water. It has, for example, been shown that when capsules of gum acacia or Encapsul® 855 containing a perfume as the non-aqueous substance are extracted continuously for 30 days at 20°C with ethanol containing no more than 1% by weight of water, no more than 15% of the total perfume originally present in the freshly prepared capsules is leached out during this period. This is totally contrary to the findings reported by The Mennen Company in GB 1 275 969 referred to earlier. This remarkable property of gum acacia is, however, not found with more conventional encapsulating materials such as Capsul starch (available from National Starch and Chemical Corp.) and $\beta$-cyclodextrin, where from 72 to 100% of the perfume is leached out by extraction with ethanol (also containing no more than 1% by weight of water) over a period of one month. It should be noted that natural gums other than gum acacia are not practical materials for spray-drying to form moisture sensitive capsules, as herein defined, as they tend to yield media which are too viscous to spray-dry at a concentration required to form intact stable capsules.

This extraction test, which will be described in greater detail later in this specification, is therefore employed as a standard test to evaluate the suitability of polymers for use according to the invention.

Preferably, the chosen polymer is one which, as capsular material permits no more than 10% by weight of the perfume to leach out under the conditions of extraction with ethanol containing no more than 1% by weight of water, as herein defined. The low-viscosity weight dextrins and gum acacia specified hereinbefore as preferred polymers possess this property

The moisture sensitive capsules will normally have an average particle size which is not greater than $100\mu m$. Usually, the capsules will possess an average particle size of from 20 to $75\mu m$, particularly when intended for use with an aerosol dispenser to avoid nozzle blockage.

The amount of moisture-sensitive capsules present in the alcoholic composition according to the invention will be from 0.1 to 20%, preferably from 0.2 to 15% by weight of the composition.

The Alcohol

The composition according to the invention will also comprise an alcohol chosen from $C_{2-6}$ alkanols and mixtures thereof. The preferred alcohols are ethanol and isopropanol.

The alcohol should contain no more than 3% by weight of water, preferably it should contain no more than 2%, most preferably no more than 1% by weight of water, to ensure that the composition itself comprises no more than 3% by weight of water.

The amount of the alcohol present in the composition according to the invention will be from 10 to 99.9%, the preferred amount depending on the product form of the composition. For example, when the composition is intended to be dispensed from an aerosol container, then the amount of alcohol will normally form from 10 to 85% by weight of the composition including the propellant normally present in such products. When the composition is intended to be dispensed from a roll-on dispenser, then the amount of the alcohol will normally form from 20 to 98% by weight of the composition. When the composition is a solid stick, then the alcohol will normally form from 10 to 80% by weight of the composition.

OPTIONAL INGREDIENTS

The alcoholic composition according to the invention can optionally comprise a suspending agent to ensure that the moisture-sensitive capsules are either maintained uniformly dispersed throughout the composition, or readily dispersable throughout the composition with minimum agitation.

When the alcoholic composition is in the form of a liquid, such as an aerosol product or a roll-on, then the suspending agent is preferably a hydrophobically-treated clay, a preferred class of which comprises smectite clays such as montmorillonites, hectorites and colloidal magnesium aluminium silicates.

Montmorillonite is colloidal, hydrated aluminium silicate obtained from bentonite of which it is the predominant constituent. A detailed discussion of bentonites can be found in the Kirk-Othmer Encyclopedia of Chemical Technology, 2nd Edition, Vol. 3 (1964) pp. 339-360, published by Interscience Publishers.

Hectorite, also a smectite clay, differs from montmorillonite in that there is almost a complete substitution of aluminium in the lattice structure of montmorillonite by magnesium and in addition, it contains lithium and fluorine.

The magnesium aluminium silicates are complexes of colloidal magnesium aluminium silicate richer in magnesium than aluminium. The hydrophobically-treated magnesium aluminium silicates are commercially available under the name VEEGUM® PRO from the R T Vanderbilt Co.

Preferred suspending agents for optional use in the invention are hydrophobic clays available under the trade name of BENTONE®. BENTONES are prepared by reacting a suitable clay in a cation exchange system with an amine. Different amines are reacted to obtain a variety of BENTONES, which may also differ in proportions of Si, MgO and $Al_2O_3$. Examples of useful BENTONE suspending agents are BENTONE®-27, which is a stearalkonium hectorite; BENTONE®-34, which is quaternium 18 bentonite; BENTONE®-38, which is quaternium 18 hectorite, all of which have a particle size of below 5 microns and are commercially available from NL Industries Inc. Other suitable BENTONE clays are BENTONE® SD1 and Bentone® SD2.

Yet further suitable clays are those hydrophobically treated smectite clays available under the trade names PERCHEM® and TIXOGEL®. Specific examples are PERCHEM® clays 44, 97 and 108 and TIXOGEL® VZ. A further class of hydrophobically-treated clays comprises hormite clays, an example of which is hydrophobically-modified attapulgite clay available under the name PERCHEM® DMA. PERCHEM® clays are sold by Perchem Limited of Harlow, Essex, Great Britain and TIXOGEL® clays by Production Chemicals Limited of Stockport, Cheshire, Great Britain.

Other useful suspending agents are fine silica powders especially the fumed silicas available commercially under the names AEROSIL® and CAB-O-SIL®, and hydroxypropyl cellulose (KLUCEL®), and other polymeric thickeners.

Mixtures of suspending agent can also be employed.

The amount of the suspending agent which can be present in the composition according to the invention will usually be within the range of from 0.2 to 20% by weight, preferably from 0.5 to 12% by weight.

The alcoholic composition according to the invention can also comprise other optional extracapsular ingredients further to enhance its properties. Examples of optional ingredients include antiperspirant and/or anticholinergic actives to enable the composition to be used as an antiperspirant, and bacteristats and/or bactericides and/or germicides, to enable the composition to be used as a deodorant. Example of antiperspirant agents, anticholinergic agents, bacteristats, bactericides and germicides are the same as those given earlier in this specification as substances included within the capsules that can provide a skin benefit.

The composition according to the invention can also optionally comprise liquefiable gaseous propellants (for aerosol products), solidifying agents such as sodium stearate, stearyl alcohol and dibenzyl sorbitol (for stick products) and emollients.

## PROCESS FOR PREPARING ALCOHOLIC COMPOSITIONS

The invention also provides a process for the manufacture of alcoholic compositions according to the invention, which process comprises the step of suspending moisture sensitive capsules in an alcohol chosen from ethanol, isopropanol or a mixture thereof, and subsequently packaging the composition so obtained.

The moisture-sensitive capsules can, as has already been stated, be prepared by conventional spray-drying techniques.

## COMPOSITION TYPE AND FORM

The alcoholic composition according to the invention is preferably a deodorant or antiperspirant composition intended for direct application to human skin. It can also be employed in body grooming products, such as aftershave products, colognes, body sprays and skin moisturisers.

The composition can be packaged in a form to suit the convenience of the consumer.

Examples of product forms of the composition are:

i) Solid sticks of the push-up or wind-up variety, each contained in a suitable container which is preferably substantially air-tight to reduce or prevent evaporation of volatile ingredients,

ii) Roll-ball applicators containing the alcoholic composition in liquid or gel form;

iii) Propellant-based aerosol products in containers containing a concentrate of the composition according to the invention together with a liquefiable gaseous propellant; and

iv) Finger-operated pump or squeeze spray containers containing the alcoholic composition according to the invention in the form of a propellant-free liquid or gel.

## Use of the Composition

The composition according to the invention is applied to human skin in a conventional manner, for example to act as a deodorant and/or an antiperspirant.

At the time of application, the moisture-sensitive capsules in the alcoholic composition should be substantially intact with not more than 15% preferably not more than 10%, and ideally not more 1% by weight of the encapsulated non-aqueous substance present in the alcoholic component of the composition.

Assuming that the skin is relatively dry at the time of application, then the moisture-sensitive capsules will remain intact, but as soon as moisture appears on the skin, for example when perspiration first appears at the skin surface, then the encapsulating special polymer will disrupt or dissolve and the non-aqueous substance contained therein will be released. When this non-aqueous substance comprises a perfume or coolant, then its presence will be apparent at least to the user of the composition, as a noticably fragrant or cooling sensation, so signalling that the composition is functioning as it is intended, as a deodorant and/or antiperspirant. When the non-aqueous substance comprises other skin benefit materials, then their release an contact with moisture will likewise enable the skin benefit to be realised.

METHOD FOR DETERMINING STABILITY IN ALCOHOL OF MOISTURE-SENSITIVE CAPSULES

Determination of Percentage Perfume Extracted from Moisture-Sensitive Capsules

Glass tubes containing approximately 1g moisture - sensitive capsules and 10g absolute ethanol (i.e. ethanol containing no more than 0.3% water) were tightly capped and kept in constant agitation for 30 days at 20°C.

Further samples with alcohol containing from 1 to 10% by weight of water were also prepared for purposes of comparison.

The liquid phase was separated from the solid moisture-sensitive capsules by filtration, so that any particulate material remaining in the filtrate made a negligible contribution to its perfume content. Millipore filters with a pore size of 0.22 $\mu$m were sufficient to retain the moisture-sensitive capsules used to provide the data herein. Filters with a smaller pore size may, however, be required to satisfy the primary condition above, if the moisture sensitive capsules contain a substantial amount of fine particulate matter. This condition is checked by dividing the first filtrate into two parts. One part is analysed for perfume content without further treatment and the second part is analyzed after an additional filtration through a 0.025 $\mu$m filter. The condition is satisfied if the perfume concentrations of the two parts of the filtrate are equal.

The amount of perfume in the separated liquid phase was determined from the peak areas obtained from Gas Chromatographic (GC) analysis. Quantification was based on the peak areas of standards containing known amounts of perfume in absolute ethanol and in each absolute ethanol/water mixture. Analysis may be based on the major peaks or the total trace (both excluding the ethanol peak). A primary condition of the analysis is that the peak areas selected for the analysis must contribute more than 50% of the total area associated with the perfume.

The percentage extraction was calculated from the concentration of perfume in the filtrate, the exact weights of moisture-sensitive capsules and absolute ethanol (or absolute ethanol/water mixture) used and knowledge of the total perfume content of the original moisture sensitive capsules.

The total perfume content of the mositure-sensitive capsules was determined by complete dissolution of a known weight of the moisture-sensitive capsules in an appropriate solvent and comparison of the GC peak areas with those for appropriate standards in the same solvent.

NOTES.

| | |
|---|---|
| 1. Weighing accuracy | - extraction ingredients ±0.01g |
| | - perfumes standards ±0.001g |
| 2. Glass tubes | - 180 mm x 13 mm internal diameter |
| | - capped with PTFE lined screw caps. |
| 3. Agitator | - Baird and Tatlock test tube rotator. |
| 4. GC Analysis Conditions | |
| | Column - 2m x 1/8" OD stainless steel |
| | Packing - 10% Carbowax 20M on Chromosorb W.HP 100/120 mesh |
| | Temperature - 60°C to 200°C at 5°C/min with final hold at 200°C for 20 mins. |
| | Carrier Gas - helium at 30 ml/min |
| | Detector - flame ionisation. |

Other suitable columns and conditions known to those skilled in the art should produce equivalent results.

When employing the forgoing method using preferred special polymers as encapsulating material, the following results were obtained:

|  | Extracting ethanol containing water (%) | Perfume extracted after 30 days (%) |
|---|---|---|
| ENCAPSUL® 855 | 0 | 10 |
|  | 1 | 14 |
|  | 4 | 54 |
|  | 5 | 61 |
|  | 10 | 85 |
| GUM ACACIA | 0 | 8 |
|  | 1 | 8 |
|  | 4 | 20 |
|  | 5 | 31 |
|  | 10 | 85 |
| CAPSUL STARCH | 0 | 71 |
|  | 1 | 67 |
|  | 4 | 92 |
|  | 5 | 93 |
|  | 10 | 103 |
| COMMERCIAL PERFUME ENCAPSULATE | 0 | 101 |
|  | 1 | 100 |
|  | 4 | 97 |
|  | 5 | 98 |
|  | 10 | 103 |

In the table above, samples extracted with ethanol containing no water or 1% by weight water were in accordance with the invention. The remaining samples extracted with ethanol containing 4%, 5% or 10% by weight of water (ie aqueous ethanol) are not in accordance with the invention, and demonstrate the inherent instability of the encapsulates when exposed to aqueous ethanol.

Also, the results obtained using Encapsul 855 and gum acacia, examples of special polymers in accordance with the invention, are contrasted with Capsul starch and a commercially available perfume encapsulate which are clearly not in accordance with the invention, in that most of the perfume encapsulated therein was extracted into the alcohol, irrespective of water content.

The foregoing method can be adapted to determine the stability in alcohol of moisture-sensitive capsules where the non-aqueous substance is a non-perfumery substance. This can be achieved by employing analytical techniques appropriate for microbiocides, skin coolants, emollients, antiperspirants, anticholinerics and other substances included in the capsules that may be leached into the extracting alcohol.

EXAMPLES

The following examples illustrate the invention.

In each case, gum acacia or Encapsul 855 was employed as the special polymer, and the encapsulate was prepared by spray drying an emulsion containing by weight polymer 28%, a perfume 7%, and water 65%.

Example 1

This example illustrates an alcoholic deodorant aerosol composition according to the invention.

| Ingredient | % w/w |
|---|---|
| Gum acacia encapsulated perfume | 1 |
| Absolute ethanol (RR Grade) | 28 |
| Bentone® 38 | 0.5 |
| Isopropyl myristate | 0.5 |
| Arcton 11 | 42 |
| Arcton 12 | 28 |
| | 100 |

## Example 2

This example illustrates an alcoholic deodorant roll-on composition according to the invention.

| Ingredient | % w/w |
|---|---|
| Gum acacia encapsulated perfume | 5 |
| Absolute ethanol (RR Grade) | 69.4 |
| Bentone® 38 | 8 |
| Dowanol DPM (DPGME) | 15 |
| Urea | 2.6 |
| | 100 |

## Example 3

This example illustrates an alcoholic deodorant stick composition according to the invention.

| Ingredient | % w/w |
|---|---|
| Gum acacia encapsulated perfume | 5 |
| Absolute ethanol (RR Grade) | 56 |
| Propylene Glycol | 36 |
| Dibenzyl sorbitol | 2 |
| Klucel® M | 1 |
| | 100 |

## Example 4

This example illustrates an alcoholic deodorant aerosol composition according to the invention having antiperspirant properties.

| Ingredient | % w/w |
|---|---|
| Gum acacia encapsulated perfume | 1 |
| Activated aluminium chlorohydrate (Antiperspirant active) | 4 |
| Ethanol containing 1% by weight water | 10 |
| Bentone® 38 | 1.5 |
| Arcton 11 | 50.3 |
| Arcton 12 | 33.2 |
| | 100 |

## Example 5

This example illustrates an alcoholic deodorant roll-on composition according to the invention having antiperspirant properties.

| Ingredient | % w/w |
|---|---|
| Gum acacia encapsulated perfume | 5 |
| Aluminium zirconium chlorohydrate (Antiperspirant active) | 25 |
| Ethanol containing 0.5% by weight water | 44.4 |
| Bentone® 38 | 8 |
| Dowanol DPM (DPGME) | 15 |
| Urea | 2.6 |
| | 100 |

Example 6

This example illustrates an alcoholic deodorant stick composition according to the invention having antiperspirant properties.

| Ingredient | % w/w |
|---|---|
| Gum acacia encapsulated perfume | 5 |
| Zirconyl aluminium glycine (Antiperspirant active) | 22 |
| Absolute ethanol (RR Grade) | 35 |
| Bentone® 38 | 5 |
| Fluid AP | 6 |
| Urea | 2 |
| Stearyl alcohol | 25 |
| | 100 |

Example 7

This example illustrates an alcoholic deodorant aerosol composition according to the invention.

| Ingredient | % w/w |
|---|---|
| Encapsul® 855 encapsulated deodorant perfume | 1 |
| Ethanol containing 0.2% by weight water | 28 |
| Bentone 38 | 0.5 |
| Isopropyl myristate | 0.5 |
| Arcton 11 | 42 |
| Arcton 12 | 28 |
| | 100 |

The deodorant perfume had the following formulation:

10

|  | % w/w |
|---|---|
| Bergamot AB 430 | 8.00 |
| p-t-Butylcyclohexyl acetate | 4.30 |
| Citronella oil | 6.00 |
| Diethyl phthalate | 8.25 |
| Ethyl vanillin | 0.20 |
| iso-Eugenol | 5.00 |
| Green Herbal AB 502 | 15.00 |
| 2-n-Heptylcyclopentanone | 0.50 |
| Indole | 1.50 |
| Inonyl formate | 5.00 |
| LRG 201 | 1.25 |
| $\alpha$-iso-Methyl ionione | 5.00 |
| $\beta$-Naphthol methyl ether | 7.50 |
| Nonanediol-1,3-diacetate | 4.00 |
| Patchouli oil | 7.00 |
| Phenylethyl phenyl acetate | 5.00 |
| Rosenta AB 380 | 6.00 |
| Sandalone | 4.00 |
| Tetrahydro muguol | 6.00 |
| $\gamma$-Undecalactone | 0.50 |
|  | 100.00 |

## Example 8

This example illustrates an alcoholic deodorant roll-on composition according to the invention.

| Ingredient | % w/w |
|---|---|
| Encapsul® 855 encapsulated menthol (coolant) | 5 |
| Absolute ethanol (RR Grade) | 69.4 |
| Bentone® 38 | 8 |
| Dowanol DPM (DPGME) | 15 |
| Urea | 2.6 |
|  | 100 |

## Example 9

This example illustrates an alcoholic deodorant stick composition according to the invention.

| Ingredient | % w/w |
|---|---|
| Encapsul 855 encapsulated menthyl pyrrolidone carboxylate (coolant) | 5 |
| Ethanol containing 2% by weight water | 56 |
| Propylene Glycol | 36 |
| Dibenzyl sorbitol | 2 |
| Klucel® M | 1 |
|  | 100 |

## Example 10

This example illustrates an alcoholic deodorant aerosol composition according to the invention having antiperspirant properties.

| Ingredient | % w/w |
|---|---|
| Encapsul® 855 encapsulated Irgasan DP300 | 1 |
| AACH* (Antiperspirant active) | 4 |
| Absolute ethanol (RR Grade) | 15 |
| Bentone® 38 | 1.5 |
| Arcton 11 | 48.3 |
| Arcton 12 | 30.2 |
| | 100 |

* Spray dried powder of a special active form of aluminium chloride prepared according to US 4 359 456. It had a particle size of <75µ and a Band III per cent aluminium value of greater than 20%.

Example 11

This example illustrates an alcoholic deodorant roll-on composition according to the invention having antiperspirant properties.

| Ingredient | % w/w |
|---|---|
| Encapsul® 855 encapsulated Benzoyl scopolamine hydrobromide | 5 |
| AACH/Urea complex° (Antiperspirant active) | 25 |
| Absolute ethanol (RR Grade) | 44.4 |
| Bentone® 38 | 8 |
| Dowanol DPM (DPGME) | 15 |
| Urea | 2.6 |
| | 100 |

° A preformed complex of Urea and a special active form of aluminium chloride (AACH-See above) prepared by codissolving AACH and urea (mole ratio 2:1) in water and spray drying the solution.

Example 12

This example illustrates an alcoholic deodorant stick composition according to the invention having antiperspirant properties.

| Ingredient | % w/w |
|---|---|
| Encapsul® 855 encapsulated perfume | 5 |
| Zirconyl aluminium glycine (Antiperspirant active) | 22 |
| Ethanol containing 3% by weight water | 35 |
| Bentone® 38 | 5 |
| Fluid AP | 6 |
| Urea | 2 |
| Stearyl alcohol | 25 |
| | 100 |

Example 13

This example illustrates an alcoholic deodorant aerosol composition according to the invention.

12

| Ingredient | % w/w |
|---|---|
| Gum acacia encapsulated perfume | 1 |
| Absolute ethanol (RR Grade) | 27.9 |
| Irgasan DP300 | 0.1 |
| Bentone® 38 | 0.5 |
| Isopropyl myristate | 0.5 |
| Arcton 11 | 42 |
| Arcton 12 | 28 |
| | 100 |

Example 14

This example illustrates an alcoholic antiperspirant roll-on composition according to the invention.

| Ingredient | % w/w |
|---|---|
| Gum acacia encapsulated perfume | 5 |
| Ethanol containing 1% by weight water | 69.35 |
| Benzoyl scopolamine hydrobromide (anticholinergic agent) | 0.05 |
| Bentone® 38 | 8 |
| Dowanol DPM (DPGME) | 15 |
| Urea | 2.6 |
| | 100 |

Example 15

This example illustrates an alcoholic deodorant aerosol composition according to the invention.

| Ingredient | % w/w |
|---|---|
| Gum acacia encapsulated perfume | 1 |
| Absolute ethanol (RR Grade) | 28.5 |
| Isopropyl myristate | 0.5 |
| Arcton 11 | 42 |
| Arcton 12 | 28 |
| | 100 |

Example 16

This example illustrates an alcoholic deodorant roll-on composition according to the invention.

| Ingredient | % w/w |
|---|---|
| Gum acacia encapsulated perfume + Irgasan DP300 | 5 |
| Ethanol containing 2% by weight water | 69.4 |
| Bentone® 38 | 8 |
| Dowanol DPM (DPGME) | 15 |
| Urea | 2.6 |
| | 100 |

Example 17

13

EP 0 303 461 B1

This example illustrates an alcoholic deodorant stick composition according to the invention.

| Ingredient | % w/w |
|---|---|
| Encapsul® 855 encapsulated perfume + menthyl lactate (coolant) | 5 |
| Ethanol containing 2.5% by weight water | 56 |
| Propylene Glycol | 36 |
| Dibenzyl sorbitol | 2 |
| Klucel® M | 1 |
| | 100 |

## Claims

1. An alcoholic composition suitable for topical application to human skin, which comprises:
   (i) from 0.1 to 20% by weight of moisture-sensitive capsules: and
   (ii) from 10 to 99.9% by weight of an alcohol chosen from $C_{2-6}$ alkanols and mixtures thereof;
   the composition comprising no more than 3% by weight of water;

   the moisture sensitive capsules having an average particle size of not greater than $100\mu m$, and comprising a non-aqueous substance encapsulated by from 40 to 90% by weight of a special polymer, the capsules being:
   (a) resistant to extraction with alcohol such that not more than 15% by weight of the non-aqueous substance originally present in the capsules is leached from the capsules during continuous extraction of the intact capsules for a period of 30 days at 20°C with ethanol containing not more than 1% by weight of water; and
   (b) sensitive to moisture such that on contact with water at the skin surface, the capsules release the encapsulated non-aqueous substance.

2. A composition according to claim 1, in which the polymer is characterised by resistance to extraction with alcohol such that not more than 10% by weight of the non-aqueous substance is leached from the capsules.

3. A composition according to claim 1 or 2, in which the polymer is a polysaccharide.

4. A composition according to claim 1, 2 or 3, in which the polymer is a dextrin.

5. A composition according to claim 4, in which the dextrin is one which, as a 50% dispersion in water has a viscosity at 25°C, using a Brookfield Viscometer fitted with an "A" type T-bar rotating at 20 rpm in helical mode, of 330 ± 20 mPas.

6. A composition according to claim 1, 2 or 3, in which the polymer is gum acacia.

7. A composition according to any preceding claim, in which the encapsulated non-aqueous substance is a perfume.

8. A composition according to claim 7, in which the perfume is a deodorant perfume.

9. A composition according to any of claims 1 to 6, in which the encapsulated non-aqueous substance is a coolant.

10. A composition according to claim 9, in which the coolant is menthol or a derivative thereof.

11. A composition according to any preceding claim, in which the non-aqueous substance is chosen from emollients, deodorants other than perfume, antiperspirant actives, anticholinergic actives and mixtures thereof.

12. A composition according to any preceding claim, in which the moisture-sensitive capsules form from

14

0.2 to 15% by weight of the composition.

13. A composition according to any preceding claim, in which the alkanol is chosen from ethanol, isopropanol and mixtures thereof.

14. A composition according to any preceding claim, further comprising a suspending agent.

15. A composition according to claim 14, in which the suspending agent is a hydrophobically-treated clay.

16. A composition according to claim 14, in which the suspending agent is hydroxypropylcellulose.

17. A composition according to claim 14, in which the suspending agent is dibenzylsorbitol.

18. A composition according to any preceding claim further comprising an antiperspirant astringent metallic salt, in addition to the moisture sensitive capsules.

19. A compositon according to claim 18, in which the astringent metallic salt is an aluminium and/or zirconium salt.

20. A composition according to any preceding claim further comprising a bactericide, in addition to the moisture-sensitive capsules.

21. A composition according to any preceding claim further comprising an anticholinergic active, in addition to the moisture sensitive capsules.

22. A process for preparing an alcoholic composition according to any of claims 1-13 which comprises the step of suspending the moisture-sensitive capsules in an alcohol chosen from ethanol, isopropanol or a mixture thereof, said composition comprising no more than 3% by weight water, and subsequently packaging the composition so obtained.

23. A process according to claim 22 for preparing an alcoholic composition according to any of claims 14-21, which comprises the s of suspending the moisture-sensitive capsules in an alcohol chosen from ethanol, isopropanol or a mixture thereof together with a suspending agent, said composition comprising no more than 3% by weight water, and subsequently packaging the composition so obtained.

**Patentansprüche**

1. Für eine örtliche Anwendung auf die menschliche Haut geeignete alkoholische Zusammensetzung, **dadurch gekennzeichnet**, daß sie
   (i) von 0,1 bis 20 Gewichtsprozent feuchtigkeitsempfindliche Kapseln und
   (ii) von 10 bis 99,9 Gewichtsprozent eines Alkohols, ausgewählt aus $C_{2-6}$-Alkanolen und Mischungen derselben,
   enthält, die Zusammensetzung nicht mehr als 3 Gewichtsprozent Wasser enthält,
   die feuchtigkeitsempfindlichen Kapseln eine durchschnittliche Teilchengröße von nicht mehr als 100 μm aufweisen und eine nichtwässerige Substanz enthalten, die durch 40 bis 90 Gewichtsprozent eines speziellen Polymeren verkapselt ist, wobei die Kapseln
   (a) gegenüber einer Extraktion mit Alkohol resistent sind derart, daß nicht mehr als 15 Gewichtsprozent der ursprünglich in den Kapseln vorhandenen nichtwässerigen Substanz aus den Kapseln während der kontinuierlichen Extraktion der intakten Kapseln für die Dauer von 30 Tagen bei 20°C mit Ethanol, enthaltend nicht mehr als 1 Gewichtsprozent Wasser, ausgelaugt werden und
   (b) empfindlich gegenüber Feuchtigkeit derart sind, daß beim Kontakt mit Wasser auf der Hautoberfläche die Kapseln die verkapselte nichtwässerige Substanz freisetzen.

2. Zusammensetzung nach Anspruch 1, **dadurch gekennzeichnet**, daß das Polymere gegenüber einer Extraktion mit Alkohol resistent ist derart, daß nicht mehr als 10 Gewichtsprozent der nichtwässerigen Substanz aus den Kapseln ausgelaugt wird.

3. Zusammensetzung nach Anspruch 1 oder 2, **dadurch gekennzeichnet**, daß das Polymere ein Polysaccharid ist.

4. Zusammensetzung nach Anspruch 1, 2 oder 3, **dadurch gekennzeichnet**, daß das Polymere ein Dextrin ist.

5. Zusammensetzung nach Anspruch 4, **dadurch gekennzeichnet**, daß das Dextrin ein solches ist, das als eine 50%ige Dispersion in Wasser unter Verwendung eines Brookfield-Viskosimeters, versehen mit einem T-Stab vom "A"-Typ, rotierend bei 20 U.p.M. in spiraliger Weise, eine Viskosität bei 25°C von 330 ± 20 mPas aufweist.

6. Zusammensetzung nach Anspruch 1, 2 oder 3, **dadurch gekennzeichnet**, daß das Polymere Akaziengummi ist.

7. Zusammensetzung nach irgendeinem der vorstehenden Ansprüche, **dadurch gekennzeichnet**, daß die verkapselte nichtwässerige Substanz ein Parfüm ist.

8. Zusammensetzung nach Anspruch 7, **dadurch gekennzeichnet**, daß das Parfüm ein Desodorans-Parfüm ist.

9. Zusammensetzung nach irgendeinem der Ansprüche 1 bis 6, **dadurch gekennzeichnet**, daß die verkapselte nichtwässerige Substanz ein Kühlmittel ist.

10. Zusammensetzung nach Anspruch 9, **dadurch gekennzeichnet**, daß das Kühlmittel Menthol oder ein Derivat davon ist.

11. Zusammensetzung nach irgendeinem der vorstehenden Ansprüche, **dadurch gekennzeichnet**, daß die nichtwässerige Substanz aus Erweichungsmitteln, anderen Desodorantien als Parfüm, aktiven Antitranspirationsmitteln, anticholinergisch aktiven Substanzen und Mischungen derselben, ausgewählt ist.

12. Zusammensetzung nach irgendeinem der vorstehenden Ansprüche, **dadurch gekennzeichnet**, daß die feuchtigkeitsempfindlichen Kapseln von 0,2 bis 15 Gewichtsprozent der Zusammensetzung ausmachen.

13. Zusammensetzung nach irgendeinem der vorstehenden Ansprüche, **dadurch gekennzeichnet**, daß das Alkanol aus Ethanol, Isopropanol und Mischungen derselben ausgewählt ist.

14. Zusammensetzung nach irgendeinem der vorstehenden Ansprüche, **dadurch gekennzeichnet**, daß sie ferner ein Suspendiermittel enthält.

15. Zusammensetzung nach Anspruch 14, **dadurch gekennzeichnet**, daß das Suspendiermittel ein hydrophob behandelter Ton ist.

16. Zusammensetzung nach Anspruch 14, **dadurch gekennzeichnet**, daß das Suspendiermittel Hydroxypropylcellulose ist.

17. Zusammensetzung nach Anspruch 14, **dadurch gekennzeichnet**, daß das Suspendiermittel Dibenzylsorbit ist.

18. Zusammensetzung nach irgendeinem der vorstehenden Ansprüche, **dadurch gekennzeichnet**, daß sie außer den feuchtigkeitsempfindlichen Kapseln ferner ein adstringierendes Antitranspirationsmetallsalz enthält.

19. Zusammensetzung nach Anspruch 18, **dadurch gekennzeichnet**, daß das adstringierende Metallsalz ein Aluminium- und/oder Zirkoniumsalz ist.

20. Zusammensetzung nach irgendeinem der vorstehenden Ansprüche, **dadurch gekennzeichnet**, daß sie außer den feuchtigkeitsempfindlichen Kapseln ferner ein Bakterizid enthält.

**21.** Zusammensetzung nach irgendeinem der vorstehenden Ansprüche, **dadurch gekennzeichnet**, daß sie außer den feuchtigkeitsempfindlichen Kapseln ferner eine anticholinergisch aktive Substanz enthält.

**22.** Verfahren zur Herstellung einer alkoholischen Zusammensetzung nach irgendeinem der Ansprüche 1 bis 13, **dadurch gekennzeichnet**, daß es die Stufe des Suspendierens der feuchtigkeitsempfindlichen Kapseln in einem aus Ethanol, Isopropanol oder einer Mischung derselben ausgewählten Alkohol, wobei die Zusammensetzung nicht mehr als 3 Gewichtsprozent Wasser enthält, und anschließendes Verpakken der so erhaltenen Zusammensetzung, umfaßt.

**23.** Verfahren nach Anspruch 22 zur Herstellung einer alkoholischen Zusammensetzung gemäß irgendeinem der Ansprüche 14 bis 21, **dadurch gekennzeichnet**, daß es die Stufe des Suspendierens der feuchtigkeitsempfindlichen Kapseln in einem aus Ethanol, Isopropanol oder einer Mischung derselben ausgewähltem Alkohol zusammen mit einem Suspendiermittel, wobei die Zusammensetzung nicht mehr als 3 Gewichtsprozent Wasser enthält, und anschließendes Verpacken der so erhaltenen Zubereitung, umfaßt.

**Revendications**

**1.** Une composition alcoolique convenant pour une application topique sur la peau humaine qui comprend :

i) de 0,1 à 20% en poids de capsules sensibles à l'humidité; et

ii) de 10 à 99,9% en poids d'un alcool choisi parmi les alcanols en $C_{2-6}$ et leurs mélanges;

la composition ne comprenant pas plus de 3% en poids d'eau;

les capsules sensibles à l'humidité ayant une taille moyenne de particule qui n'est pas supérieure à 100$\mu$m, et comprenant une substance non-aqueuse encapsulée par de 40 à 90% en poids d'un polymère spécial, les capsules étant :

(a) résistantes à une extraction par l'alcool de telle manière que pas plus de 15% du poids de la substance non-aqueuse présente à l'origine dans les capsules ne soient éliminés par lessivage des capsules au cours d'une extraction en continu des capsules intactes pendant une période de 30 jours à 20°C par de l'éthanol ne contenant pas plus de 1% en poids d'eau; et

(b) sensibles à l'humidité de telle manière qu'au contact avec de l'eau à la surface de la peau, les capsules libèrent la substance encapsulée non-aqueuse.

**2.** Une composition selon la revendication 1, dans laquelle le polymère est caractérisé par une résistance à l'extraction par un alcool telle que pas plus de 10% en poids de la substance non-aqueuse n'est lessivé hors des capsules.

**3.** Une composition selon la revendication 1 ou 2, dans laquelle le polymère est un polysaccharide.

**4.** Une composition selon la revendication 1, 2 ou 3, dans laquelle le polymère est une dextrine.

**5.** Une composition selon la revendication 4, dans laquelle la dextrine est une dextrine qui, sous forme d'une dispersion à 50% dans l'eau a une viscosité à 25°C, en utilisant un Viscosimètre Brookfield équipée d'un barreau en T de type "A" tournant à 20tpm dans le mode hélicoïdal, de 330 ± 20 mPas.

**6.** Une composition selon la revendication 1, 2 ou 3, dans laquelle le polymère est la gomme acacia.

**7.** Une composition selon l'une quelconque des revendications précédentes, dans laquelle la substance non-aqueuse encapsulée est un parfum.

**8.** Une composition selon la revendication 7, dans laquelle le parfum est un parfum déodorant.

**9.** Une composition selon l'une des revendications 1 à 6, dans laquelle la substance non-aqueuse encapsulée est un agent de refroidissement.

**10.** Une composition selon la revendication 9, dans laquelle l'agent de refroidissement est le menthol ou un dérivé de celui-ci.

**11.** Une composition selon l'une quelconque des revendications précédentes, dans laquelle la substance non-aqueuse est choisie parmi les émollients, les déodorants autres que des parfums, les actifs antiperspirants, les actifs anticholinergiques et des mélanges de ceux-ci.

**12.** Une composition selon l'une quelconque des revendications précédentes, dans laquelle les capsules sensibles à l'humidité forment de 0,2 à 15% en poids de la composition.

**13.** Une composition selon l'une quelconque des revendications précédentes, dans laquelle l'alcanol est choisi parmi l'éthanol, l'isopropanol et des mélanges de ceux-ci.

**14.** Une composition selon l'une quelconque des revendications précédentes, comprenant de plus un agent de mise en suspension.

**15.** Une composition selon la revendication 14, dans laquelle l'agent de mise en suspension est une argile traitée de manière hydrophobe.

**16.** Une composition selon la revendication 14, dans laquelle l'agent de mise en suspension est l'hydroxypropylcellulose.

**17.** Une composition selon la revendication 14, dans laquelle l'agent de mise en suspension est le dibenzylsorbitol.

**18.** Une composition selon l'une quelconque des revendications précédentes, comprenant de plus un sel métallique astringent antiperspirant, en plus des capsules sensibles à l'humidité.

**19.** Une composition selon la revendication 18, dans laquelle le sel métallique astringent est un sel d'aluminium et/ou de zirconium.

**20.** Une composition selon l'une quelconque des revendications précédentes, comprenant de plus un bactéricide, en plus des capsules sensibles à l'humidité.

**21.** Une composition selon l'une quelconque des revendications précédentes, comprenant de plus un actif anticholinergique, en plus des capsules sensibles à l'humidité.

**22.** Un procédé pour préparer une composition alcoolique selon l'une quelconque des revendications 1-13 lequel comprend l'étape de mise en suspension des capsules sensibles à l'humidité dans un alcool choisi parmi l'éthanol, l'isopropanol ou un mélange de ceux-ci, ladite composition ne comprenant pas plus de 3% en poids d'eau, et ensuite un conditionnement de la composition ainsi obtenue.

**23.** Un procédé selon la revendication 22 pour préparer une composition alcoolique selon l'une quelconque des revendications 14-21, lequel comprend l'étape de mise en suspension des capsules sensibles à l'humidité dans un alcool choisi parmi l'éthanol, l'isopropanol ou un mélange de ceux-ci conjointement avec un agent de mise en suspension, ladite composition ne comprenant pas plus de 3% en poids d'eau, et ensuite un conditionnement de la composition ainsi obtenue.